# EUROPEAN PATENT APPLICATION

(11) **EP 3 097 849 A1**
(43) Date of publication of application: **30.11.2016**
(21) Application number: 15425036.9
(22) Date of filing: 26.05.2015
(51) Int. Cl.: A61B 5/053

(54) **EYEGLASSES FOR MONITORING ONE OR MORE PHYSIOLOGICAL PARAMETERS OF THE USER THAT WEARS THEM**

(71) Applicant: Akern S.r.L., 50065 Pontassieve (FI) (IT)
(72) Inventor: Talluri, Antonio, 50012 Bagno A Pipoli (FI) (IT)
(74) Representative: Emmi, Mario

(57) **Abstract**

The present invention concerns a device characterized in that it is in the shape of eyeglasses (1) or of a mask and comprising first means to measure one or more physiological parameters of the user.

## Description

### Technical field

The present invention refers to the technical field relative to the new and emerging technology called "wearable", that is all those portable and wearable technologies, modelled around the body of the person, whose body is used as a natural support to their functioning as well as the object of observation, measurement and therapy.

In particular, the invention refers to an innovative device configured to be wearable as clothing or an accessory for the head or the face of a user, for example in the shape and with the functions of multifunctional glasses, mask, bandana, helmet, etc. (therefore a "wearable" or "smart" device), integrating in *sé* one or contemporarily more different functions to allow the measurement and/or monitoring of one or more physiological parameters.

### Background art

Various apparatus of very reduced size are known that allow, each one of them, to detect, measure or monitor one or more physiological parameters.

For example, a wrist bracelet/watch has been recently proposed in commerce which, once worn, allows to detect electrical parameters such as the bio-impedance, in such a way as to obtain a body hydration state or estimate the calories together with the heart rate.

Such a product, for example, can be visualized at the following webpage:
http://www.bbc.com/news/technology-30681002

The bracelet/watch is therefore capable of injecting a current through two electrodes and detecting a voltage drop in such a way as to obtain bioelectrical impedance analysis values.

Such a bracelet/watch is particularly useful and has been studied precisely for sports use, since a monitoring of the correct hydration state is fundamental to complete a sports performance in the best way (be it a training session or a competition).

Other sensors, singularly known, allow to monitor other parameters such as the heart beat (heart rate monitor), the body temperature (thermocouples), the oxygenation of blood (pulse oximetry), the acceleration, the equilibrium, etc.

All these parameters can be useful both in phase of sports activity and in phase of normal rest or everyday life. Such parameters result to be even more necessary in the presence of chronic diseases to monitor the patient.

For instance, in the obstructive bronchopathy (BPCO) it is advantageous to verify as often as possible the oxygen saturation state of the red blood cells (transcutaneous pulse oximetry or SpO₂).

Naturally, each one of such (and eventual others) physiological parameters requires the use of separated devices, which result to be hardly found and applied in a comfortable way for the user, above all during the normal everyday activities, be them of clinical, sports or other nature.

Also the use of a so-made bracelet/watch for the determination of the hydration states suffers from some technical inconvenience.

Being applied to the wrist, it is often subject to sudden movements of acceleration, lifting/lowering that can alter in an acute manner the physiological state of the body segment on which the measurement is made, altering in an acute manner the measure itself, with a confusing result.

The stability of the sensor itself is precarious and therefore also the quality of the signal intercepted by a sensor on a bracelet that cannot be perfectly stable results to be imprecise or subject to artifacts.

In fact, if to avoid rotations or slidings it was tightened around the segment, the duress or compression would alter the normal physiology (localized ischemia, localized capillary compression, etc.) of the tissues, thus distorting the measure.

In that sense, therefore, not only does the measure of the hydration state detected result to be not always precise, but also in the case in which other sensors were also integrated for other measurements, these would not result to be sufficiently precise and repetitive.

Last, in order to read the data, the display of the watch/bracelet has to be drawn near the eyes, also due to the size of the characters that is necessarily small. This operation is certainly not comfortable, above all for people with the low vision.

### Disclosure of invention

It is therefore the aim of the present invention to provide an innovative device that solves said technical inconveniences.

In particular, it is the aim of the present invention to provide a device that, in a precise and comfortable way for the user and with a highly repetitive and stable positioning, allows to detect one or more physiological parameters.

These and other aims are therefore reached with a device 1, as per claim 1.

Such a device is configured to be wearable as clothing or accessory for the head or the face of a user, for example in the shape or with the functions of eyeglasses, mask, bandana and foresees first means to allow the measurement and/or monitoring of one or more physiological parameters.

In particular, in an advantageous manner, such a device is in the shape of eyeglasses or of a mask or of a simple "bandana" (elastic band around the head) and is characterized in that it comprises such first means to allow the measurement of one or more physiological parameters of the user that wears them.

The eyeglasses, as well as the bandanas, have the advantage of being of common use and of easy realization. Each sportsman, in general, wears protection eyeglasses and often also "bandanas". For example, both in running and by bicycle sunglasses are used, which have also a protective function from powders and gnats (therefore not necessarily with corrective lenses or lenses shielding the light).

Also non sports people, during everyday life, wear or can wear eyeglasses without bother for the user.

The positioning of the eyeglasses has a fixed confirmation, very stable and therefore all the measures detected are less subject to artifacts and therefore more trustable and repetitive. The same can be said for the "bandana" band or for a mask that anyway results to be well stable on the face.

Last, the eyeglasses, thanks to their structure, can easily integrate the various components necessary for their functioning and can lodge a display that is continuously well visible.

Further advantages can be deduced from the dependent claim.

### Brief description of drawings

Further features and advantages of the present device, as per the invention will result to be clearer with the description that follows of some embodiments, made to illustrate but not to limit, with reference to the annexed drawings, wherein:
- Figure 1 shows in an axonometric view eyeglasses in accordance with the present invention;
- Figure 2 is an internal front view, both of an arm of the frames and of the front part of the frames in order to highlight the electrodes that inject the current and those that detect the consequent voltage drop;
- Figure 3 is a schematization of the components further necessary to detect the bioelectrical impedance analysis parameters;
- Figure 4 and figure 5 show a solution in which the eyeglasses foresee one or more displays 20 for visualizing the parameters detected;
- Figure 6 shows a solution in which the eyeglasses integrate also an artificial horizon;
- Figure 7 schematizes a transfer of the data detected to a mobile telephony device for visualizing them.
- Figure 8 shows very adherent eyeglasses of circular shape suitable for detecting signals also from the forehead and from the temples (for example, cyclist eyeglasses) and therefore easily provided simultaneously with multiple sensors, disseminated at area in the frame and in the arms, such as photoplethysmography for Spo2, impedance, thermocouples, etc.

### Description of some preferred embodiments

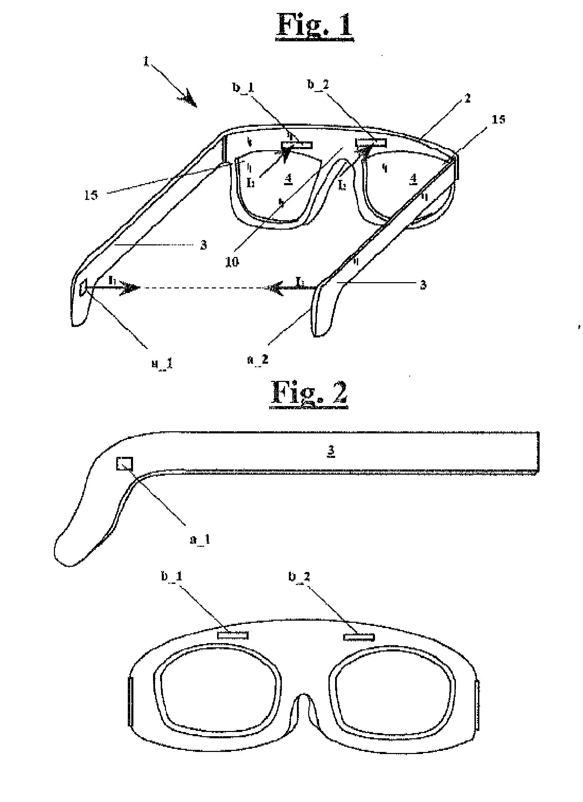

Figure 1 shows eyeglasses 1 in accordance with the present invention.

This, as it is well known, foresees frames 2 that include a front part where the lenses 4 are fixed and conformed to rest on the bridge of the nose of the user. Such frames foresee, as usual, the two arms 3 destined to rest in the space between back of the ears and the junction of the same to the head.

In accordance with the present invention, such eyeglasses can be of any nature, that is for seeing, for the sun, for resting, for sports activities and therefore even without prescripted and/or blackened lenses but only for protection purposes from powders and also masks, for example diving ones.

Having said that, such eyeglasses integrate first means configured to allow to detect one or more physiological parameters of the person who wears them and described in detail below.

The first element present between said first means concerns, in accordance with the tetra-polar technique, specific electrodes (a_1 a_2; b_1, b_2), voltmeter (V) and generator (G) for the detection of one or more electrical parameters (generally called bioelectrical impedance analysis ones) such as impedance Z, or resistance R, reactance and phase.

The scientific articles are numerous that discuss the detection of bioelectrical impedance analysis parameters in order to obtain through them, with well-known formulas, physiological parameters relative to lean mass, fat mass and body hydration state.

The known technique, as tetra-polar measure, consists in placing two electrodes, in general single-use and auto-adhesive, distally on the back of the hand and of the foot, ipsilateral, to which is connected a generator of alternate current, kept constant or of known constant, and preferably sinusoidal signal with frequencies that vary between 1 kHz and 1000 kHz. The most diffused frequency is 50 kHz. The values of the most diffused constant current oscillate between 100 and 800 micro amperes.

Inside this system of injection two fixed electrodes are placed that, connected to a voltmeter, permit to measure the voltage present (therefore evaluate the current circulating in the body following the injection of a first constant current or of known reference), allowing in such a manner to obtain the bioelectrical impedance analysis values of the segment comprised between them (impedance **Z,** resistance **R,** reactance **Xc,** phase **ϕ**).

Having said that, in accordance with the invention, the electrodes result to be set into the frames in a convenient position.

For example, in a non limiting case, the injectors can be applied in the peri-auricular area, as a pair of electrodes injecting a first current according to standard values of the tetra-polar technique described above (for example, frequency of 50 kHz and 200 microamperes of constant or known intensity).

Further two electrodes can, for example, be placed in the lateral supra-orbital area, connected to a voltmeter. In such a way, they detect the voltage drop so that a processor **(P)** connected to the voltmeter can interpolate the known formulas to obtain an instantaneous hydration state of the tissues.

The arms 3 can naturally be an ideal location also for the necessary electronic components.

Figure 2, for that purpose, schematizes in a front view one of the two arms 3 to highlight an electrode (a_1) responsible for the injection of a first current (I₁). Always figure 2 shows in a front view the front part of the frames (the part that holds the lenses 4) to highlight the two receiving electrodes (b_1, b_2). As described above, the two receiving electrodes connect to a voltmeter (V) (not shown in this figure) to obtain an impedance value Z. Just for descriptive simplicity purposes, in figure 2 also the second arm on which the electrode a_2 is found has been omitted.

The locations of the electrodes have been represented in accordance with what has been described above, that is in correspondence of the arms 3 in peri-auricular position for the injecting electrodes (a_1, a_2) and in lateral supra-orbital position for the receiving electrodes (b_1, b_2) even if, obviously, other locations can be foreseen and without for this moving apart from the present inventive concept.

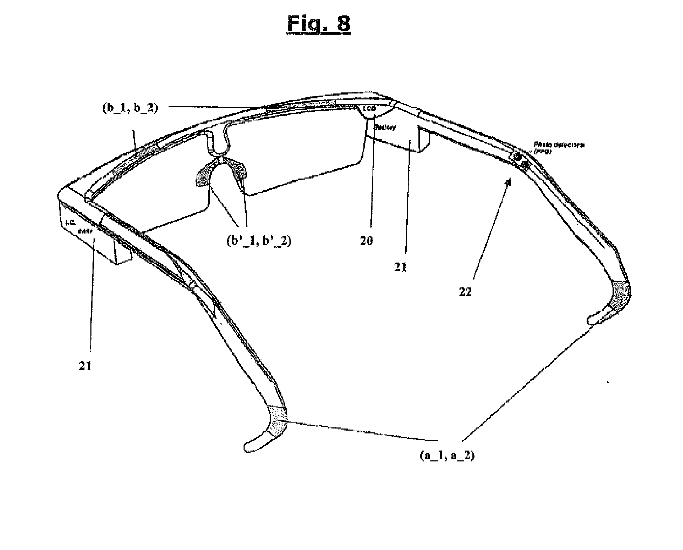

Figure 8 shows a type of eyeglasses quite diffused in the sports field and that results to be very adherent to the face. This has the advantage of allowing an arrangement of a plurality of sensors of various nature both in the front part and in that of the temples in such a way as to detect with precision as many physiological parameters as possible.

Precisely figure 8 shows the two electrodes (a_1, a_2) in peri-auricular position and the two electrodes (b_1, b_2) in lateral supra-orbital position for the receiving electrodes.

Going back to figure 1, just for clarity purposes, also such a figure schematizes such electrodes and highlights the two injecting electrodes (a_1, a_2) that inject a first current (**I**₁). The current circulates in the section between the electrodes (a-b) and therefore is dephased, modifying its intensity to a value (I₂). The receiving electrodes (b_1, b_2), connected to the voltmeter, measure the voltage drop and from here, for example through a specific processor, one can obtain the electric measure of the impedance Z and the physiological parameters connected to it (for example, hydration state).

In combination with or alternatively to the voltmeter, systems can be foreseen to have sensitivity to the phase and that calculate the components of the impedance vector Z, that is resistance R, reactance xc and phase ϕ.

As said, the electrodes, both the injecting ones and the receiving ones, not necessarily have to be positioned in the positions represented in figure 2 or figure 8. For example, noting would exclude the possibility of setting the receiving electrodes (b_1, b_2) in correspondence of the area of support of the frames on the nasal support, as shown in figure 8. In this case, the electrodes have been indicated with the denomination (b'_1, b'_2) and are to be intended as substitutive of the preceding ones (b_1, b_2).

Naturally, always inside the frames, the further electronics (processor P, batteries, etc.) has to be present that allows, as said, to obtain the hydration value and/or other physiological parameters derivable from the knowledge of the bioelectrical impedance analysis parameters that can be obtained in accordance with the tetra-polar technique.

A possible location, naturally, is that relative always to one or both the arms, or also a symmetry position in correspondence of the support point of the frames on the nose (position indicated in figure 1 with number 10).

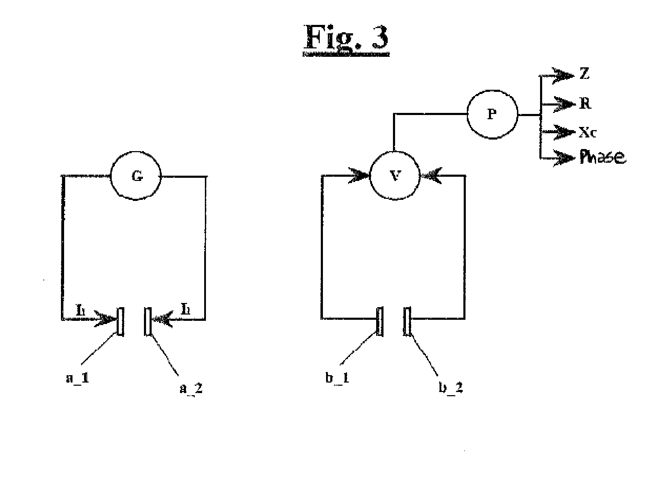

It can be schematized, as shown in figure 3, the positioning of the generator (G) connected to the electrodes (a 1, a_2) for the injection of the current (I₁) and the voltmeter (V) which, being connected to the receiving electrodes (b_1, b_2) measures the voltage drop and, thanks to the processor P, detects the bioelectrical impedance analysis parameters and from them the relative physiological parameters.

The same processor P can be programmed to eventually extrapolate, if necessary, other measures deriving from the other eventual sensors present.

Figure 8 shows in correspondence of the arms a containment box 21 into which the batteries are lodged and the eventual electronics necessary, for example voltmeter, generator, processor.

Going on with the structural description of the invention, the eyeglasses of figure 1 can further include, or alternatively, other sensors set in the structure (therefore arm or other parts of frames) such as a photo-sensor to detect the oxygen saturation.

Pulse oximetry is founded on the absorbency features of the red and infra-red light of oxygenated and deoxygenated hemoglobin. The oxygenated hemoglobin absorbs mostly the infrared light and permits a greater passage of red light. The hemoglobin with minor content of oxygen absorbs more the red light and permits a greater massage to the infrared light. The red light is in the strip of light of wavelength 600-750 nm. The infrared light is in the strip of light of wavelength 850-1000 nm. Variations of reflection intensity are also connected to the greater or minor reflecting blood volume, therefore permitting to obtain simultaneously also an evaluation of volume variations (photoplethysmographic) and therefore the heart beat.

The sensors are in general applied on quite transparent areas with a good capillary bend, as is found in the periorbital or periauricular area.

Having said that, the pulse oximetry/photoplethysmograph, as it is well known in the state of the art, is constituted by a light emitter with red and infrared LED that shines (illuminates) a quite transparent site and is rich with blood, while the light emitted is reflected by the blood and is detected by a photodetector, collimated.

For example, in figure 8 with number 22 such photodetectors are shown.

The data detected such as heart beat, temperature, oximetry and other eventual ones are rendered visible on specific second means that can be, for example, in the shape of a display 20, such as liquid crystals, and for instance placed in the high areas placed at the angles on top of the glasses (positions 15 of figure 1). In such a manner, without obstructing the normal visual of the lenses, the user can see different physiological parameters measured.

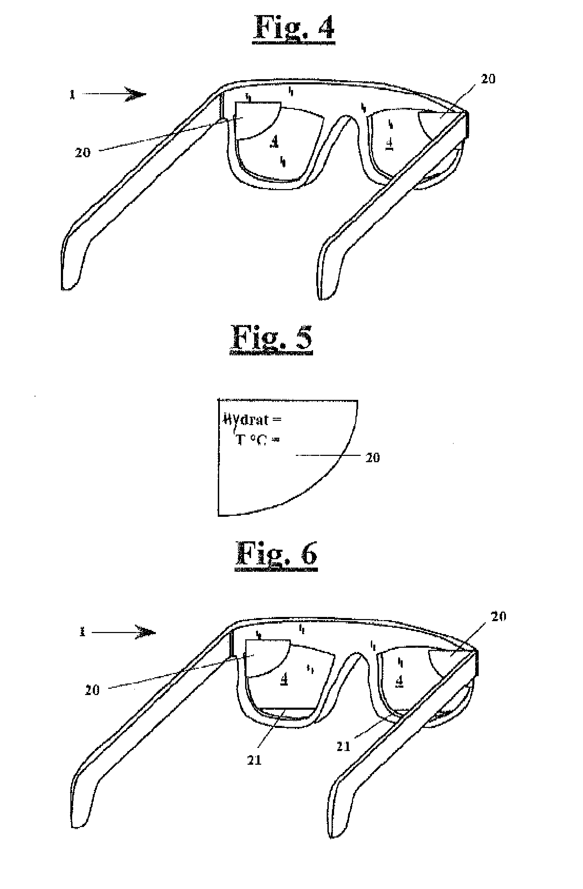

Figure 4 schematizes such a display 20 which is placed in electronic communication with said first means to detect the physiological parameters in such a way as to shown them visually (one or more than one of them).

Figure 5, just as a way of a non limiting example, shows the display 20 that indicates a hydration state and a temperature.

Both the photo-plethysmograph and the thermometer are known in the state of the art and for that reason are not further described in detail here. In that case, they are set in the structure of the eyeglasses.

Going on with the structural description of the invention, such a typology of eyeglasses can integrate other components suitable for determining a physiological state of the user.

An artificial horizon 21 can be further integrated, as also shown in figure 6.

The artificial horizon is placed in the visual area 4 at the bottom and this, just to make an example, can result to be much more useful, for example during the navigation since it represents a fixed reference for those who suffer seasickness, therefore making the suffering become lighter. It could be simply composed of a liquid that is rendered free to oscillate in a double hermetic glass, or a led artificial horizon, assisted and operated electronically with triaxial accelerometer of the known art and commonly diffused in the smartphones.

Independently from the means integrated in the eyeglasses that would allow the measurement of one or more physiological parameters, the eyeglasses can also integrate a system of wireless communication with a mobile telephone of new generation or with other screen electronic apparatus such as tablets, pcs and the like, for monitoring from remote or for data saving.

Through a wireless connection (for example, in a non limiting manner a Bluetooth or WIFI connection), the eyeglasses are connected to a specific application (APP) in such a way as to show on video the measures detected, rather than highlighting them or requiring the presence of a display integrated in the eyeglasses.

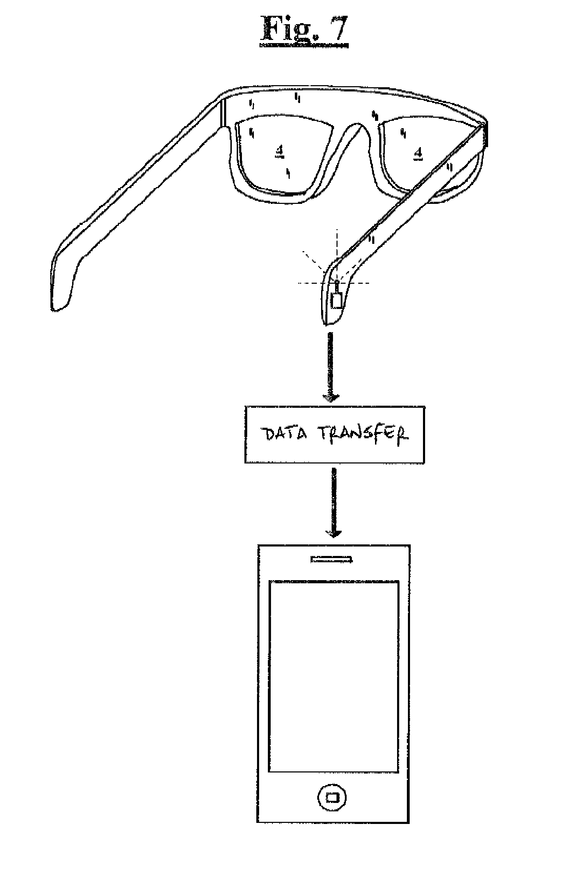

Figure 7 schematizes this solution and highlights precisely the connection of the eyeglasses with a mobile telephony device to which the data detected is transferred.

In use, therefore, the user wears the eyeglasses normally and operates the first means, for example through a single bottom that allows to feed such first means with a normal battery such as that of watches.

At this point, the various parameters are detected and visualized on the integrated screen 20 or on the display of a mobile telephony device.

What has been described until now is naturally valid for the mask and the bandana.

The bandana, in particular, can be realized with a specific material, such as fabric, rubber, etc., in order to be able to contain the components described above.

## Claims

1. A device **characterized in that** it has the shape of eyeglasses (1) or of a mask or of a bandana and comprising first means to measure one or more physiological parameters of the user .

2. A device, as per claim 1, wherein said first means are configured to measure at least one or more of the following physiological parameters:
- Bioelectrical impedance analysis parameters, such as impedance and/or resistance and/or reactance and/or phase;
- Heart rate;
- Oxygen saturation in the blood;
- Temperature;
- Equilibrium/Posture.

3. A device, as per claim 1 or 2, wherein said first means comprise:
- At least a pair of electrodes (a_1, a_2) for injecting a current (I₁);
- At least a pair of electrodes (b_1, b_2) for measuring a voltage drop as a consequence of the current (I₁) injected;
- Means of measurement (V, P) to obtain a measure of one or more bioelectrical impedance analysis parameters.

4. A device, as per claim 3, wherein the device is a pair of eyeglasses and said pair of electrodes (a_1, a_2) is positioned in the arms (3) in peri-auricular position, while the receiving electrodes (b_1, b_2) are arranged in supra-orbital or temporal position.

5. A device, as per one or more of the preceding claims, wherein an absorbency or light reflection device is foreseen, to detect the oxygen saturation and the heart rate.

6. A device, as per one or more of the preceding claims, wherein a device is foreseen configured to detect the body temperature.

7. A device, as per one or more of the preceding claims, wherein the eyeglasses or the mask is provided with lenses (4) and wherein said lenses are furnished with an artificial horizon.

8. A device, as per claim 7, wherein said artificial horizon is generated through a liquid interposed in a double glass forming the lens of the eyeglasses or of the mask.

9. A device, as per claim 7, wherein said artificial horizon is generated through a led assisted and operated electronically with triaxial accelerometer.

10. A device, as per one or more of the preceding claims, wherein second means (20) are foreseen to visualize said parameters.

11. A device, as per claim 10, wherein said second means to visualize said parameters comprise at least a display (20) integrated in the eyeglasses or in the mask.

12. A device, as per one or more of the preceding claims, wherein connection means are foreseen to put in communication said device with a screen device, such as a mobile telephony device or a PC.

13. A device, as per claim 12, wherein said connection means are of the wireless type.

14. A device, as per claim 12 or 13, wherein said connection means foresee a Bluetooth or WIFI communication.
